# EUROPEAN PATENT SPECIFICATION

(11) **EP 3 060 274 B1**
(45) Date of publication and mention of the grant of the patent: **04.12.2019**
(21) Application number: 14792986.3
(22) Date of filing: 24.10.2014
(51) Int. Cl.: A61M 5/168, A61M 5/172, A61M 39/22

(54) **MEDICAL FLUID INJECTION MANIFOLD**
MEDIZINISCHER FLÜSSIGKEITSINJEKTIONSVERTEILER
COLLECTEUR D'INJECTION DE FLUIDES MÉDICAUX

(30) Priority: 25.10.2013 US 201361895934 P
(43) Date of publication of application: 31.08.2016
(73) Proprietor: Acist Medical Systems, Inc., Eden Prairie, MN 55344 (US)
(72) Inventor: COOLIDGE, Troy M., Victoria, MN 55386 (US); PFEFFER, Derek, Plymouth, MN 55441 (US); GOLDBERGER, Mattie L., Crystal, MN 55422 (US)
(74) Representative: Pistolesi, Roberto
(86) International application number: PCT/US2014/062235
(87) International publication number: WO 2015/061723

(56) References cited:
- WO-A1-00/16849
- WO-A1-2013/043868
- WO-A2-2010/039662
- US-A1- 2003 181 850
- US-A1- 2005 077 225
- US-A1- 2011 071 390
- US-A1- 2011 071 465
- US-A1- 2014 107 480

## Description

### TECHNICAL FIELD

This disclosure relates to fluid manifolds and, more particularly, to fluid manifolds for powered medical fluid injection devices.

### BACKGROUND

Medical fluid injection devices are typically used to inject medical fluid into a patient. These devices often include one or more reservoirs to hold the medical fluid and one or more pressurizing units to inject the medical fluid into the patient. For example, a contrast media powered injection device may include a reservoir containing contrast medium and another reservoir containing a diluent, such as saline. The contract media powered injection device may have a syringe that is used to inject the contrast media into the patient and another syringe or pump that is used to inject the diluent into the patient. When used, the contrast media and/or diluent is typically injected from the powered injection device into a catheter inserted into the patient. The contrast media injection device may be used during certain medical procedures, such as an angiographic or computed tomography (CT) procedure.

In some applications, a patient's hemodynamic pressure may be monitored during an injection procedure. For example, during angiography, a health care provider may record the intravascular and intra-cardiac pressures of the patient between injections of high pressure contrast media. To monitor the hemodynamic pressure of the patient during an injection procedure in such applications, a pressure sensor may be placed in fluid communication with the catheter inserted into the patient. The pressure sensor may sense the hemodynamic pressure of the patient through a column of fluid extending through the catheter to the pressure sensor. Fluid communication between the catheter and the pressure sensor may be closed during high pressure fluid injection to shield the pressure sensor from high pressures developed during contrast injection. Fluid communication may be reopened, however, when high pressure contrast media is not being injected through the catheter.

Ensuring that a pressure sensor accurately measures a patient's hemodynamic pressure during an injection procedure may be useful for the safe and efficacious use of an injection device. While sophisticated, high-resolution pressure sensors can be used to obtain highly accurate pressure data, pressure sensors in contrast injection devices are typically disposed of after a single patient procedure to avoid cross-contamination between patients. This generally precludes the use of expensive, high-resolution pressure sensors. Appropriately implementing a comparatively lower resolution pressure sensor within a contrast injection system may be useful to ensure that the pressure sensor provides sufficiently accurate data.

A medical fluid injection manifold according to the preamble of claim 1 is know from document WO 2010/039662 A2.

### SUMMARY

In general, this disclosure relates to a fluid injection manifold for a contrast injection device. That fluid injection manifold includes a pressure sensor configured to measure a hemodynamic pressure of a patient. The fluid injection manifold may be a disposable component or part of a disposable single-use patient kit that is replaced on a contrast injection device between each injection procedure. The fluid injection manifold may provide different fluid pathways for routing different medical fluid from the contrast injection device to a tubing system extending between the contrast injection device and a catheter inserted into a patient. For example, the fluid injection manifold may have a first fluid pathway through which saline is delivered to a patient and a second fluid pathway through which contrast media is delivered to the patient.

To control the flow of contrast media and saline between the contrast injection device and the patient and to also control fluid communication between the patient and the pressure sensor, the fluid injection manifold may have valves positioned between fluid pressurizing units of the contrast injection device and the tubing system extending to the patient. The pressure sensor may be positioned between the valves so that the pressure sensor can be isolated from pressurized fluid flowing through the manifold yet also be placed in selective fluid communication with a patient line to measure a patient's hemodynamic pressure. For example, the pressure sensor may be placed in selective fluid communication with a fluid lumen of the tubing system through which diluent is injected into the patient during an injection procedure. The diluent may provide a low viscosity fluid column through which the patient's hemodynamic pressure is communicated. This may provide a more accurate pressure signal than if the patient's hemodynamic pressure was communicated through a fluid column containing high viscosity contrast media. Moreover, in instances where a relatively uniform diluent composition is used across different patient procedures, pressure signal artifacts associated with the diluent can be compensated for when determining a patient's hemodynamic pressure.

In one example, a medical fluid injection manifold is described that includes a manifold housing and a pressure sensor. The manifold housing has a first inlet port configured to receive a first pressurized medical fluid, a second inlet port configured to receive a second pressurized medical fluid, a first outlet port configured to discharge the first pressurized medical fluid, and a second outlet port configured to discharge the second pressurized medical fluid. The pressure sensor is configured to measure a hemodynamic pressure of a patient. According to the example, the pressure sensor is positioned within the manifold housing and in selective fluid communication with the first outlet port.

In another example, a medical fluid injection system is described that includes a medical fluid injection device, a tubing system, a fluid injection manifold, and a pressure sensor. The medical fluid injection device includes a first fluid pressurizing unit in fluid communication with a first medical fluid and a second fluid pressurizing unit in fluid communication with a second medical fluid. The tubing system includes a first fluid lumen configured to convey the first medical fluid from the medical fluid injection device to a patient and a second fluid lumen configured to convey the second medical fluid from the medical fluid injection device to the patient. The fluid injection manifold includes a manifold housing having a first inlet port configured to receive the first medical fluid from the first fluid pressurizing unit, a second inlet port configured to receive the second medical fluid from the second fluid pressurizing unit, a first outlet port configured to discharge the first medical fluid from the manifold housing into the first fluid lumen, and a second outlet port configured to discharge the second medical fluid from the manifold housing into the second fluid lumen. The pressure sensor is positioned within the fluid injection manifold and configured to measure a hemodynamic pressure of the patent. According to the example, the pressure sensor is in selective fluid communication with a column of fluid extending from the patient through the first fluid lumen.

In another example, a method is described that includes setting a first valve and a second valve of a fluid injection manifold housing to a first operational position such that a first pressurized medical fluid is injectable through the fluid injection manifold housing while a second pressurized medical fluid is blocked from flowing through the fluid injection manifold housing. The example method also includes setting the first valve and the second valve of the fluid injection manifold housing to a second operational position such that a second pressurized medical fluid is injectable through the fluid injection manifold housing while the first pressurized medical fluid is blocked from flowing through the fluid injection manifold housing. The example method further includes setting the first valve and the second valve of the fluid injection manifold housing to a third operational position such that both the first pressurized medical fluid and the second pressurized medical fluid are blocked from flowing through the fluid injection manifold housing but a pressure sensor positioned within the fluid injection manifold is allowed to communicate with a column of fluid extending from a patient into the fluid injection manifold housing.

The details of one or more examples are set forth in the accompanying drawings and the description below. Other features, objects, and advantages will be apparent from the description and drawings, and from the claims.

### BRIEF DESCRIPTION OF DRAWINGS

FIG. 1 is a perspective drawing of an example powered medical fluid injection system.
FIG. 2 is a perspective drawing of an example configuration of a fluid injection manifold that can be used in the medical fluid injection device of FIG. 1.
FIGS. 3A and 3B are drawings illustrating one example type of valve that can be used in the fluid injection manifold of FIG. 2.
FIG. 4 is a cross-sectional illustration of the fluid injection manifold of FIG. 2 illustrating an example configuration by which a pressure sensor and dampening device can be positioned within the manifold.
FIGS. 5-9 are conceptual illustrations showing different operational positions that can be assumed by valves in the fluid injection manifold of FIG. 2 to control fluid movement through the manifold.

### DETAILED DESCRIPTION

The following detailed description is exemplary in nature and is not intended to limit the scope, applicability, or configuration of the invention in any way. Rather, the following description provides practical illustrations for implementing exemplary embodiments of the present invention. Examples of constructions, materials, dimensions, and manufacturing processes may be provided for selected elements, and all other elements employ that which is known to those of skill in the field of the invention. Those skilled in the art will recognize that many of the examples provided have suitable alternatives that can be utilized.

A powered medical fluid injector may be used to inject contrast media into the body of a patient during a diagnostic imaging procedure. The contrast media may contain an active contrast agent that interacts with radiation or electromagnetic energy from a diagnostic imaging machine to enhance the visual contrast of structures or fluids within the body of the patient, for example, as compared to structures or fluids not exposed to the contrast agent. For example, the contrast agent may highlight features that would otherwise be less distinguishable from nearby tissue to help a clinician diagnose and treat a patient's medical condition. The powered medical fluid injection may also be used to inject a diluent (e.g. saline) into the body of the patient during the diagnostic imaging procedure. The diluent may be injected simultaneously with the contrast media, for example to dilute the contrast media, or between contrast media injections, for example to flush the catheter of contrast agent.

In addition to injecting medical fluids into the body of a patient during a medical procedure, the powered medical fluid injector may also be used to monitor a hemodynamic pressure of the patient during the injection procedure. For example, during angiography, a health care provider may record the intravascular and intra-cardiac pressures of the patient between injections of high pressure contrast media. The health care provider may look for pressure values falling within the general range of -1 psi to +6 psi (-51.7 mmHg to 310 mmHg) to confirm the hemodynamic health of the patient. To monitor the hemodynamic pressure of the patient during such procedures, the patient may be connected to a pressure sensor that is in fluid communication with an injection catheter inserted into the patient. The pressure sensor may be located in or adjacent to the medical fluid injection device and be in fluid communication with the patient catheter via a patient tubing set extending between the injection device and the catheter.

As will be described in greater detail below, this disclosure generally relates to a fluid manifold for a medical fluid injection device that includes a pressure sensor. The fluid manifold, which may be referred to as a fluid cassette or a patient-specific tubing set, is generally configured to be detachably attached to the medical fluid injection device. The fluid manifold may or may not be discarded after a single patient use and replaced with a new, sterile fluid manifold for a subsequent patient injection procedure. The fluid manifold can control fluid routing and flow between the medical fluid injection device and a catheter inserted into the patient. For example, the fluid manifold may include one or more valves that control fluid flow from a contrast media pressurizing device and/or a diluent pressurizing device to the catheter. The one or more valves of the fluid manifold may also control fluid flow between the catheter and the pressure sensor, for example, to measure the patient's hemodynamic pressure between high pressure injections of contrast media.

In some examples, the fluid manifold has separate inlet ports and outlet ports to separately route contrast media and diluent through the manifold without mixing the medical fluids. For example, the fluid manifold may have two outlet ports that are separately connectable to different fluid lumens extending between the manifold and the catheter. The different fluid lumens can allow the diluent and contrast media to be delivered to the patient through separate fluid pathways, for example, until the fluid pathways converge at or near the catheter of the patient. Such an arrangement can allow the catheter to be flushed, for example by injecting diluent through the dedicated fluid pathway and into the catheter, without needing to push contrast media residing in the fluid pathway into the patient, as may otherwise be required if the contrast media and diluent traveled through a common fluid lumen. In addition, the dedicated diluent and contrast media fluid lumens may facilitate more accurate pressure monitoring than if a pressure sensor were connected to a fluid lumen through which both contrast media and diluent were injected.

For instance, in one example, the fluid manifold is configured to allow selective fluid communication between a pressure sensor contained within the fluid manifold and a fluid lumen through which diluent is injected into a patient during an injection procedure. During injection of high pressure contrast media, a valve of the fluid manifold may close fluid communication between the diluent fluid line and the pressure sensor. This can prevent high pressure from traveling from the patient catheter and through diluent contained in the diluent fluid lumen, which may otherwise damage the pressure sensor. On the other hand, the valve of the fluid manifold may open to allow fluid communication between the diluent fluid line and the pressure sensor when it is desired to measure the hemodynamic pressure of the patient. The hemodynamic pressure of the patient may travel through the patient catheter and through diluent contained in the diluent fluid lumen to be sensed by the pressure sensor. This may provide a comparatively low viscosity fluid pathway through which patient hemodynamic pressure can translate, for example, providing higher resolution pressure measurements than if the pressure were to translate through a fluid lumen filled with comparatively viscous contrast media. Of course, in other examples, the hemodynamic pressure of the patient can be monitored through a column contrast media contained in a contrast media lumen.

In some additional examples, the tubing extending between the fluid manifold of the injection device and the patient catheter is designed to help avoid excitation frequencies that may cause the pressure sensor to generate artificial pressure signals. For example, during some patient pressure fluctuations, such as high frequency left ventricle ejection, the pressure change within the patient may cause the tubing line extending between the patient and the pressure sensor to resonate with a certain excitation frequency corresponding to the pressure change. If the excitation frequency corresponds to the resonance frequency of the tubing line, the tubing line itself can cause a pressure sensor to generate an artificial pressure signal, potentially obscuring clinically significant pressure data. Accordingly, in some examples, the resonance frequency of the tubing line is increased so that most, if not all, typically experienced by a human patient are slower than the resonance frequency.

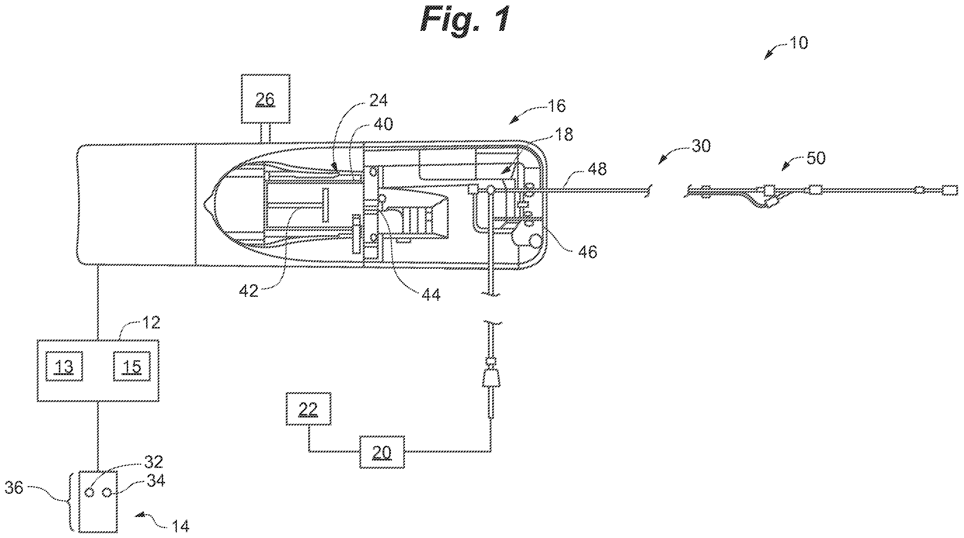

FIG. 1 is a perspective drawing of one example of a powered medical fluid injection system 10 that may include a fluid injection manifold through which fluid is supplied to a patient during an injection procedure, such as an angiogram. In the example of FIG. 1, injector system 10 includes a main console 12, a hand held remote control 14, a medical fluid injection device 16, and a fluid injection manifold 18. Medical fluid injection device 16 includes a first fluid pressurizing unit 20 fluidly connected to a first reservoir 22 of medical fluid and a second fluid pressurizing unit 24 fluidly connected to a second reservoir 26 of medical fluid. In particular, medical fluid injection device 16 is illustrated as having a first fluid pressurizing unit 20 that is a peristaltic pump and a second fluid pressurizing unit that is a syringe. In operation, first fluid pressurizing unit 20 and second fluid pressurizing unit 24 draw fluid from first reservoir 22 and second reservoir 26, respectively, and inject the fluid through manifold 18 into a tubing 30 extending between the injection device 16 and a catheter (not illustrated) inserted into a patient. Powered medical fluid injection system 10 is only one example of a configuration of a powered injector that can be used in accordance with the disclosure and the disclosure is not limited in that respect. For example, injector system 10 may include a syringe fluidly connected to first reservoir 22 for injecting the fluid in lieu of the peristaltic pump illustrated in FIG. 1. As another example, injector system 10 may include other types of positive displacement pumps in addition to or in lieu of those illustrated in FIG. 1.

As described in greater detail below, fluid injection manifold 18 may be removably connected to medical fluid injection device 16. The fluid injection manifold may contain inlet ports and outlet ports for conveying medical fluid from first reservoir 22 and second reservoir 26, respectively, to tubing 30. In addition, the fluid injection manifold 18 may include a pressure sensor that is configured to be placed in selective fluid communication with fluid in tubing 30. During an injection procedure, the pressure sensor can measure a hemodynamic pressure of a patient when medical injection device 16 is not operating to inject high pressure contrast media into the patient.

Console 12 in the example of FIG. 1 houses various electrical controls for medical fluid injection device 16, such as one or more processors, computer readable memory, and motor(s) which power first fluid pressurizing unit 20 and/or second fluid pressurizing unit 24. Console 12 may include a user interface and a display through which a user may enter control settings and monitor the operational state of system 10. For example, to use powered medical fluid injection system 10, an operator may interact with console 12 to set up various parameters and/or protocols to be used for a given injection procedure. The operator may interact with console 12 to enter injection parameters for flow rate, maximum injection volume, maximum injection pressure, rise time, or other parameters. In one embodiment, console 12 includes a touch-screen panel.

Remote control 14 can be connected to console 12 by a cable (although in other examples remote control 14 may be connected by a wireless connection such as an RF, infrared, optic, or ultrasonic link). Remote control 14 is, in the example of FIG. 1, a handheld control which includes reset and diluent push button switches 32 and 34, respectively, and a flow rate control lever or trigger 36. By squeezing trigger 36, the operator can provide a command signal to console 12 to control the rate at which medial fluid is discharged from the first pressurizing unit 20 and/or second pressurizing unit 24 and injected into tubing 30 connected to a patient catheter. In other examples, system 10 does not include remote control 14. In these examples, a user may control the operation of system 10 directly via console 12.

To control the operation of fluid injection system 10, the fluid injection system includes a processor 13 and memory 15. Processor 13 can control the filling and discharge of a first medical fluid from first pressurizing unit 20 and a second medical fluid from second pressurizing unit 24 with the aid of instructions associated with program information stored in memory 15. Processor 13 may also control the filling and discharge of medical fluid based on instructions received from a user, e.g., via console 12 and/or hand held remote control 14. Instructions executed by processor 13 may, for example, define fluid delivery programs that specify the quantity, rate, and/or pressure with which medical fluid is to be delivered from first pressurizing unit 20 and/or second pressurizing unit 24 into a patient catheter during a diagnostic imaging procedure. Instructions executed by processor 13 may also control the opening and closing of valves within system 10, such as valves to fill the pressurizing units of injection device 16 and valves controlling fluid movement through injection manifold 18.

Processor 13 of medical fluid injection system 10 may include one or more processors, such as one or more microprocessors, digital signal processors (DSPs), application specific integrated circuits (ASICs), field programmable gate arrays (FPGAs), programmable logic circuitry, or the like, either alone or in any suitable combination. In general, processor 13 may receive electrical signals from input devices such as console 12 and remote control 14 and provide electrical signals to output devices, such as valves, motors, and a display. For example, processor 13 may provide signals to a display associated with console 12 that cause the display to display operation data, alerts, status information, and operator prompts. As another example, processor 13 may provide signals to valves connected to injection manifold 18 to control the opening and closing of the valves.

Memory 15 may store instructions and related data that, when executed by processor 13, cause contrast injection system 10 and processor 13 to perform the functions attributed to them in this disclosure. For example, memory 15 of injection system 10 may store instructions for execution by processor 13 including, e.g., commands for actuating valves, instructions for filling and/or discharging the pressurizing units, instructions for monitoring and comparing a signal generated by a pressure sensor contained within injection manifold 18, and any other information regarding the system 10.

First pressurizing unit 20 in FIG. 1 is capable of pumping fluid from first reservoir 22 through injection manifold 18 and into tubing 30. First pressurizing unit 20 may draw a first medical fluid from first reservoir 22 and discharge the fluid under an increased pressure out into a first inlet tube 38 connecting the fluid pressurizing unit to injection manifold 18. When first pressurizing unit 20 is implemented as a pump, the pump may be an axial pump, a centrifugal pump, a pusher plate pump, a piston-driven pump, or other pumping device. In one such example, the pump is a squeeze pump that squeezes a compressible fluid tube (e.g., a plastic tube) in a controlled manner, e.g., such as a peristaltic pump, to progressively pressurize and move medical fluid through the tube. In still other examples, first pressurizing unit 20 may be implemented as a syringe, e.g., so that medical fluid injection device 16 is a dual syringe injector.

Second pressurizing unit 24 is also capable of pumping fluid through injection manifold 18 into tubing 30 leading to a patient catheter. In the example of FIG. 1, second pressurizing unit 24 is illustrated as a syringe pump that includes a syringe barrel 40 and a syringe plunger 42. The syringe barrel 40 receives and holds a medical fluid from second reservoir 26. Syringe plunger 42 is disposed within and moveable relative to the syringe barrel. To fill the syringe, the syringe may be fluidly coupled to second reservoir 26 and syringe plunger 42 driven to its furthest forward position adjacent a discharge outlet 44. This can expel the majority of the air that is located within the syringe. Thereafter, the syringe plunger 42 is retracted within the syringe barrel, creating a vacuum within the syringe barrel that draws medical fluid from second reservoir 26 into syringe barrel 40. To subsequently discharge the medical fluid, fluid communication between syringe barrel 40 and second reservoir 26 is closed, and syringe plunger 42 is advanced forward in the syringe barrel to pressurize and discharge the medical fluid from the syringe barrel. The pressurized medical fluid is expelled into injection manifold 18, e.g., via a second inlet tubing line connecting discharge outlet 44 to the manifold (not illustrated in FIG. 1). In other examples, fluid injection system 10 does not include second reservoir 26 but may instead utilize a prefilled syringe that is inserted into a syringe holder of second pressurizing unit 24.

First pressurizing unit 20 and second pressurizing unit 24 draw fluid from first reservoir 22 and second reservoir 26, respectively, and discharge pressurized fluids through injection manifold 18 into tubing 30. First reservoir 22 can contain a first medical fluid, such as a diluent (e.g., saline, water) or a fluid medication. Second reservoir 26 can contain a second medical fluid, such as a contrast media. Contrast media is a liquid that can be injected into a patient to highlight selected areas of the patient while the patient is being scanned, e.g., radiographically. Contrast media typical has a viscosity ranging from approximately 1 centipoise to approximately 50 centipoise (e.g., from approximately 4 centipoise to approximately 30 centipoise) and, in some examples, may have an organically (i.e., non-ionic) or non-organically (i.e., ionic) bound molecule that functions to provide contrast, such as organically or non-organically bound iodine. The viscosity of the contrast media is typically greater than that of a diluent, which may have a viscosity less than 1 centipoise.

In various examples, first reservoir 22 and second reservoir 26 can be a bag, bottle, syringe, or other container capable of storing fluid. In some cases, one or both of the first reservoir 22 and the second reservoir 26 may be fabricated from glass while, in other cases, one or both may be fabricated from plastic. In addition, in some cases, first reservoir 22 and/or second reservoir 26 may be designed to hang from a corresponding reservoir hook. The fluid contained with first reservoir 22 and second reservoir 26 may be connected to first pressurizing unit 20 and second pressurizing unit 24, respectively, via tubing sets that extend between each reservoir and an inlet or fill port of a respective pressurizing unit.

During operation, first pressurizing unit 20 pressurizes a first medical fluid (e.g., diluent) to generate a pressurized first medical fluid that is discharged into injection manifold 18. In addition, second pressurizing unit 24 pressurizes a second medical fluid (e.g., contrast media) to generate a pressurized second medical fluid that is discharged into injection manifold 18. Typically, first pressurizing unit 20 and second pressurizing unit 24 will operate in alternating sequence, e.g., to inject contrast media into a patient followed by a saline flush. In other examples, however, first pressurizing unit 20 and second pressurizing unit 24 can operate simultaneously to inject both the first medical fluid and the second medical fluid into a patient, e.g., substantially simultaneously. Such a configuration may be useful to generate a mixture of the first medical fluid and the second medical fluid that is then injected into the patient.

Independent of the specific operating sequence of first pressurizing unit 20 and second pressurizing unit 24, the pressurizing units inject medical fluids through injection manifold 18. Injection manifold 18 is fluidly connected to tubing 30 and can route fluid from the pressurizing units to the tubing. In general, tubing 30 provides one or more fluid lumens extending between medical fluid injection device 16 and a catheter inserted into a patient. For example, tubing 30 may provide a junction inside of a housing of injection device 16 that merges a fluid pathway connected to the first pressurizing unit 20 with a separate fluid pathway connected to the second pressurizing unit 24. In such an example, tubing 30 may provide a single fluid lumen extending between injection device 16 and a patient catheter through which both first and second medical fluids (e.g., diluent and contrast media) travel during operation of the injector. Alternatively, tubing 30 may have multiple fluid lumens extending from injection device 16 through which fluid from first pressurizing unit 20 and fluid from second pressurizing unit 24 can separately travel during operation of the injector. Such a configuration can provide dedicated fluid pathways for the different medical fluids injected by powered medical fluid injection system 10.

In the example of FIG. 1, tubing 30 is illustrated as having a first fluid lumen 46, a second fluid lumen 48, and a junction 50. First fluid lumen 46 is in fluid communication with first pressurizing unit 20 via injection manifold 18. Second fluid lumen 48 is in fluid communication with second pressurizing unit 24 via injection manifold 18. Unlike a tubing configuration that provides a shared fluid lumen through which both first and second medical fluids travel, tubing 30 provides separate fluid lumens through which first and second medical fluids can be separately conveyed to a patient. Such an arrangement can allow a catheter connected to tubing 30 to be flushed, for example by injecting diluent through first fluid lumen 46 and into the catheter, without needing to push contrast media residing in the fluid pathway into the patient, as may otherwise be required if the contrast media and diluent traveled through a common fluid lumen. In addition, in some examples, a pressure sensor contained in injection manifold 18 can be placed in selective fluid communication with one fluid lumen (e.g., first fluid lumen 46 containing diluent) rather than another fluid lumen (e.g., second fluid lumen 48 containing contrast media) to monitor a patient's hemodynamic pressure. In instances where one fluid lumen contains a medical fluid that translates pressure better than the medical fluid in another fluid lumen, this arrangement can lead to more accurate pressure measurements by the pressure sensor.

In examples in which tubing 30 has multiple fluids lumens, the multiple fluid lumens may extend between injection manifold 30 and a patient catheter. In one example, tubing 30 provides separate fluid lumens extending an entire distance between injection manifold 30 and a patient catheter. The separate fluid lumens may merge into a single, common lumen at the catheter or connect to a multi-lumen catheter. In another example, such as the example illustrated in FIG. 1, the separate lumens of tubing 30 merge into a common lumen at a junction 50 prior to connecting to a patient catheter. When so configured, junction 50 can be positioned at any suitable location between injection manifold 18 and a patient catheter. In some examples, junction 50 is positioned closer to a patient than to medical injection device 16, such that a distance between the patient and the junction is less than a distance between the junction and the injection device. For example, junction 50 may be located less than 1 meter (m) from a patient, such as less than 0.5 m, less than 0.25 m, less than 0.1 m, or less than 0.05 m. Positioning junction 50 close to a patient may minimize the length of the tubing through which both medical fluids travel in a common lumen, e.g., to provide catheter flushing and pressure measurement benefits noted above. Of course, in other examples, tubing 30 may not have multiple fluid lumens or may have a shared fluid lumen that is longer in length than the separate fluid lumens.

Although not illustrated in FIG. 1, injection device 16 may also include an air detector. Tubing conveying fluid from the first pressurizing unit 20 and/or second pressurizing 24 can pass through the air detector, which is capable of detecting air bubbles or air columns within the tubing. If the air detector detects a measureable or otherwise significant amount of air within the tubing, the detector may generate an alarm signal for injection device 16. In such a case, a warning or alarm message may be displayed to the operator on console 12, indicating that air has been detected. In addition to or in lieu of generating a warning or alarm message, injection device 16 may automatically pause, or terminate, a fluid injection procedure if an air detector has detected air in the tubing, such that the air is not delivered to the catheter of the patient.

Because medical fluid injection device 16 may be used for many injections and patient procedures, the components of the injection system can potentially become contaminated with patient fluid during a specific patient injection procedure and may need to be replaced after each patient procedure. This may include tubing 30 extending between injection device 16 and the patient catheter as well as a pressure sensor associated with the injection device and other tubes connecting first pressurizing unit 20 and second pressurizing unit 24 to the patient catheter. Replacing potentially contaminable components after each patient procedure helps ensure the sterility of the injection environment and avoids cross-contamination from one patient to another patient.

Fluid injection manifold 18 can contain patient-specific tubing and a pressure sensor used to measure a specific patient's hemodynamic pressure that are intended to be replaced after each patient injection procedure. To help facilitate quick and accurate replacement of the disposable components between patient injection procedures, fluid injection manifold 18 can be designed to be removable from injection device 16 and replaceable. In the example, of FIG. 1, fluid injection manifold 18 is illustrated as being insertable into a housing of injection device 18, where the manifold can be connected therein. In other examples, fluid injection manifold 18 may be detachably connected to an external surface of fluid injection device 16. Regardless, fluid injection manifold 18 can be removed from fluid injection device 16 after a patient injection procedure and replaced with a new, sterile manifold for a subsequent injection procedure.

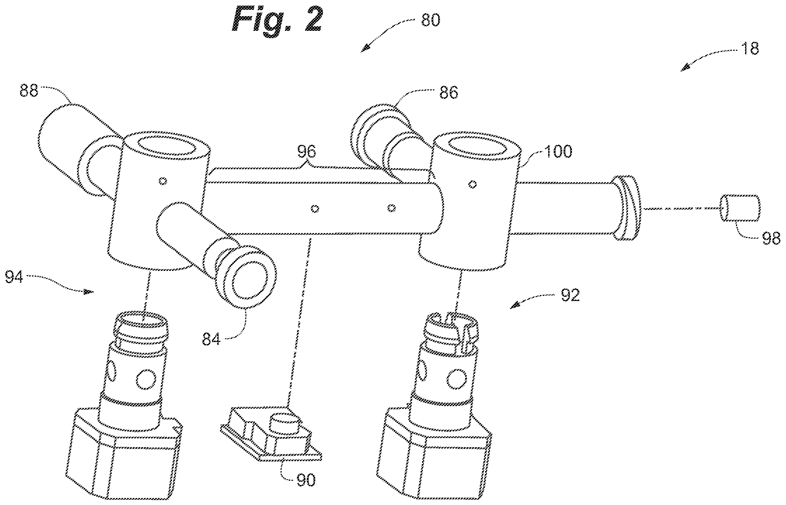

FIG. 2 is a perspective drawing of one example configuration of fluid injection manifold 18 that can be used in the medical fluid injection device 16 in FIG. 1. In the illustrated example, injection manifold 18 has a manifold housing 80 that defines fluid pathways through which a first medical fluid discharged from first pressurizing unit 20 (FIG. 1) and a second medical fluid discharged from second pressurizing unit 24 can travel before entering tubing into a patient catheter via tubing 30. In particular, manifold housing 80 in FIG. 2 is illustrated as having a first inlet port 82 configured to receive a first pressurized medical fluid (e.g., diluent from first reservoir 22 in FIG. 1) and a second inlet port 84 configured to receive a second pressurized medical fluid (e.g., contrast media from second reservoir 26 in FIG. 1). Manifold housing 80 also has a first outlet port 86 through which medical fluid entering the manifold via first inlet port 82 discharges and a second outlet port 88 through which medical fluid entering via second inlet port 84 discharges.

In use, injection manifold 18 can be inserted into fluid injection device 16 (FIG. 1) and fluidly connected to the fluid pressurizing devices of the injection device. For example, manifold housing 80 can be positioned inside of or adjacent to a housing of fluid injection device 16 and first inlet port 82 and second inlet port 84 connected to fluid discharge points from first pressurizing unit 20 and second pressurizing unit 24, respectively, via tubing (not illustrated on FIG. 2). In addition, first outlet port 86 and second outlet port 88 can be connected to tubing 30 (FIG. 1), for example by connecting tubing 30 directly to the outlet ports or by utilizing intermediate tubing segments that extend between the first and second outlet ports and tubing 30. In one example, first outlet port 86 is connected to first fluid lumen 46 of tubing 30 and second outlet port 88 is connected to second fluid lumen 48 of tubing 30.

To help enable quick and accurate replacement of a patient pressure sensor between patient injection procedures, fluid injection manifold 18 also includes a pressure sensor 90. Pressure sensor 90 is illustrated in FIG. 2 as being located outside of but insertable into manifold housing 80. When installed, pressure sensor 90 is positioned within manifold housing 80 and configured to measure the pressure of a fluid within the manifold housing. For example, when manifold housing 80 is connected to a patient line (within a patient), pressure sensor 90 is capable of functioning as a hemodynamic monitor for the patient. The pressure sensor 90 can sense the hemodynamic pressure of the patient through a column of fluid that extends through the patient line (e.g., catheter), through tubing 30, and through one or both of outlet ports 86 and 88. In one example, pressure sensor 90 is a pressure transducer that converts detected fluid pressures into electrical signals that may be monitored or otherwise used by medical fluid injection device 16 and/or another monitoring device associated with injection system 10.

During operation of injection manifold 18, contrast media may be delivered through manifold housing 80 at a high pressure (e.g., a pressure around 1200 pounds per square inch) via a fluid pathway extending between second inlet port 84 and second outlet port 88. By contrast, diluent may be injected through manifold housing 80 via a fluid pathway extending between first inlet port 82 and first outlet port 86 at a comparatively lower pressure, such as a pressure less than 125 pounds per square inch. Depending on the design of pressure sensor 90, the pressure in manifold housing 80 during contrast media injection may be higher than the pressure rating of the pressure sensor. Accordingly, to prevent damage of pressure sensor 90, the pressure sensor may be shielded from high pressures developed during contrast media injection by a fluid delivery valve. The fluid delivery valve may close fluid communication between pressure sensor 90 and a patient catheter during comparatively high pressure contrast media injection and open fluid communication between the pressure sensor and the patient catheter when high pressure contrast media is not being injected in to the patient.

To shield pressure sensor 90 from high pressures generated during contrast injection, injection manifold 18 includes at least one valve which, in the example of FIG. 2 is illustrated as two valves: first valve 92 and second valve 94. First valve 92 controls fluid communication between pressure sensor 90 and a fluid pathway extending between first inlet port 82 and first outlet port 86. Second valve 94 controls fluid communication between pressure sensor 90 and a fluid pathway extending between second inlet port 84 and second outlet port 88. First valve 92 can provide selective fluid communication between first inlet port 82, first outlet port 86, and pressure sensor 90. Second valve 94 can provide selective fluid communication between second inlet port 84, second outlet port 88, and pressure sensor 90.

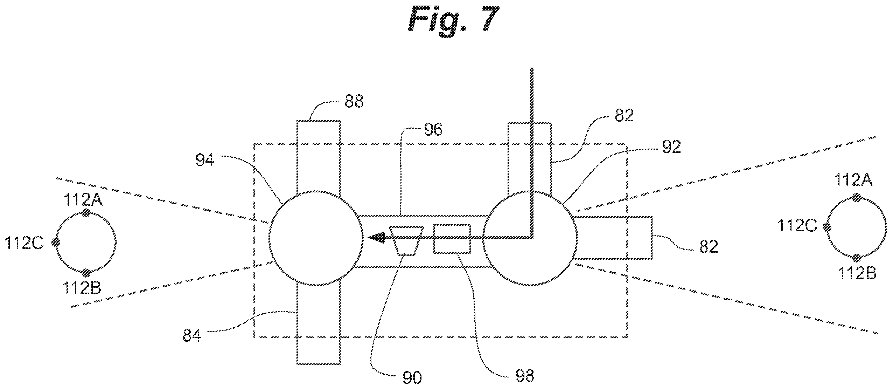

Pressure sensor 90 can be located at any suitable position within manifold housing 80 that enables the pressure sensor to be shielded from high pressures generated during contrast injection yet allows the pressure sensor to sense a patient's hemodynamic pressure, e.g., when high pressure injection is not taking place. In the example of FIG. 2, manifold housing 80 includes a bridge portion 96 extending between first valve 92 and second valve 94. Pressure sensor 90 is located within bridge portion 96, such that the pressure sensor can sense the pressure of fluid contained within or traveling through the bridge portion. Pressure sensor 90 can be pressure isolated by closing first valve 92 and second valve 94. However, opening first valve 92 can place the pressure sensor in pressure communication with first inlet port 82 and/or first outlet port 86. Likewise, opening second valve 94 can place the pressure sensor in pressure communication with second inlet port 84 and/or second outlet port 88. For example, as described below with respect to FIG. 7, first valve 92 may be opened to place pressure sensor 90 in pressure communication with a column of fluid entering manifold housing 80 from a patient via first outlet 86. This may enable the pressure sensor to detect the patient's hemodynamic pressure via the column of fluid.

While pressure sensor 90 is illustrated as being positioned within bridging portion 96 of manifold housing 80, the pressure sensor can be positioned in any suitable location that allows the pressure sensor to be selectively closed to pressure communication with a patient line during high pressure injection yet opened to pressure communication to measure hemodynamic pressure. For instance, in other examples, manifold housing 80 does not have a bridging portion extending between first valve 92 and second valve 94. In these examples, manifold housing 80 may have a dead end stem extending from first valve 92 that is not coupled to second valve 94. Pressure sensor 90 can be positioned within the dead end stem portion of the manifold housing and placed in selective fluid communication with first outlet 86 via first valve 92. Other manifold housing configurations are possible, and it should be appreciated that the disclosure is not limited in this respect.

As noted above, pressure sensor 90 can be placed in pressure communication with a patient line inserted into a patient, when high pressure contrast media is not flowing through the patient line. The hemodynamic pressure of the patient can act on fluid within the patient line and translate through fluid within tubing 30 back to pressure sensor 90. When pressure changes in the patient occur, the pressure changes can cause pressure waves that communicate through liquid extending from the patient to pressure sensor 90, allowing the pressure sensor to detect the pressure changes at a remote location outside of the patient.

In some instances, pressure fluctuations within the patient may cause pressure sensor 90 to detect pressure spikes that overwhelm the pressure sensor and/or obscure clinically relevant pressure data. For example, high frequency pressure fluctuations within a patient may cause pressure to change in tubing 30 (FIG. 1) at a frequency at or near the resonance frequency of the tubing. When this occurs, the tubing may oscillate and cause pressure sensor 90 to generate pressure signal artifacts that are not associated with actual pressure changes in the patient but rather changes in the measurement system, e.g., due to tubing oscillation.

To help improve the accuracy of hemodynamic pressure measurements made by pressure sensor 90, fluid injection manifold 18 may include a dampening device that dampens fluid signals (e.g., pressure waves) translating from a patient to the pressure sensor. In the example of FIG. 2, fluid injection manifold 18 is illustrated as having a fluid signal dampening device 98. Dampening device 98 is shown positioned outside of manifold housing 80 but insertable into the housing. Upon insertion, dampening device 98 is positioned within bridging portion 96 between pressure sensor 90 and first valve 92.

Dampening device 98 can function to dampen pressure signals communicated from a patient to pressure sensor 90 via a column of liquid extending between the patient and the sensor. For example, dampening device 98 can reduce the magnitude of pressure detected by pressure sensor 90 such that if the pressure sensor were to detect a pressure of a certain magnitude without dampening device 98, the pressure sensor will detect a pressure of a lesser magnitude with dampening device 98, under the same pressure conditions. Such a feature can be useful, for example, to dampen pressure spikes that would otherwise overwhelm pressure sensor 90 to a level that is readable by the pressure sensor. That being said, in other examples, injection manifold 18 does not include a dampening device.

When used, dampening device 98 can be implemented using any suitable feature that can dampen a fluid signal communicated from a patient line to pressure sensor 90. In one example, dampening device 98 is a filter through which liquid traveling from a patient tube must pass before reaching pressure sensor 90. The filter may have one or more porous openings that provide a reduced cross-sectional flow path as compared to when the filter is not present in manifold housing 80. For example, the filter may have a pore size less than 100 microns, such as less than 50 microns, less than 20 microns, or less than 10 microns.

In addition to or in lieu of utilizing dampening device 98, tubing 30 (FIG. 1) connected to injection manifold 18 may be configured to help reduce resonant excitation that can lead to false pressure readings. In general, the resonant frequency of injection system 10 and, more particularly tubing 30, is dependent on the stiffness and mass of the components exposed to pressure fluctuations. Further, stiffness is dependent on the bulk modulus of the system, which can be a combination of the bulk modulus of the fluids in the system and the bulk modulus of the fluid containing structures (e.g., tubing 30) in the system.

In some examples, the resonance frequency of injection system 10 and tubing 30 is increased by increasing the stiffness of the tubing and/or decreasing the fluid volume held in the system, which may help elevate the resonance frequency above a pressure change frequency typically experienced during an injection procedure. For example, an internal diameter of tubing 30 (e.g., first lumen 46 and/or second lumen 48) can be reduced and/or a length of tubing 30 shortened to increase the stiffness of the tubing system and reduce the volume of fluid held in the tubing system. In one example, pressure sensor 90 measures a patient's hemodynamic pressure via a diluent (e.g., saline) line rather than a contrast media line, which can have a smaller internal diameter and corresponding higher resonance frequency than the contrast media line.

Independent of the specific tubing structure or configuration through which pressure sensor 90 measures a patient's hemodynamic pressure, in some examples, the tubing and/or system has a bulk modulus greater than 20 ksi (thousand pounds per inch), such as greater than 25 ksi, greater than 30 ksi, or greater than 35 ksi. Depending on the configuration, the tubing and/or system may have a resonance frequency greater than approximately 20 Hz, such as greater than approximately 22 Hz, or greater than approximately 25 Hz.

Fluid injection manifold 18 in FIG. 2 includes inlet ports 82, 84 and outlet ports 86, 88. In general, the inlet ports 82, 84 are openings through which fluid can enter manifold housing 80 and outlet ports 86, 88 are openings through which fluid can exit the manifold housing. Inlet ports 82, 84 and outlet ports 86, 88 provide access to fluid pathways within manifold housing 80, such as tubing or ducts, which bound movement of fluid through the housing to certain defined locations. Inlet ports 82, 84 and/or outlet ports 86, 88 can have mechanical engagement features for connecting flexible tubing to the ports to create fluid-tight connections. Examples of suitable mechanical engagement features include, but are not limited to, luer lock fittings, threaded connectors, friction fit connectors, and the like.

Manifold housing 80 can have any suitable number and configuration of inlet ports and outlet ports. The number and configuration of the inlet ports and outlet ports of manifold housing 80 may depend, for example, on the number of fluid pressurizing units provided by fluid injection device 16 (FIG. 1) and on the number of fluid lumens associated with tubing 30. In some examples, each inlet port and outlet port of manifold housing 80 has a same cross-sectional area or diameter. In other examples, at least one port of manifold housing 80 has a different cross-sectional area or diameter than at least one other port of the housing. For instance, in one example, first outlet port 86 has a smaller cross-sectional internal diameter than second outlet port 88. For example, first outlet port 86 and/or first fluid lumen 46 may have an internal diameter less than 0.075 inches, such as less than 0.065 inches, or between approximately 0.025 inches and approximately 0.060 inches. By contrast, second outlet port 88 and/or second fluid lumen 48 may have an internal diameter greater than 0.075 inches, such as greater than 0.08 inches, or between approximately 0.085 inches and approximately 0.1 inches. The foregoing sizes are merely examples, and other sizes are possible.

In the example of FIG. 2, inlet ports 82, 84 and outlet ports 86, 88 each extend distally away from a valve stem housing portion of manifold housing 80. In particular, first inlet port 82 extends outward away from a first valve stem housing 100 and is co-axially aligned with bridge portion 96, such that first inlet port 82 is on an opposite side of first valve stem housing 100 from bridge portion 96. First outlet port 86 extends outward away from first valve stem housing 100 and is orthogonally aligned with first inlet port 82 and bridge portion 96. Second inlet port 84 extends outward away from a second valve stem housing 102 and is orthogonally aligned with bridge portion 96. Second outlet port 88 also extends outward away from second valve stem housing 102 and is co-axially aligned with second inlet port 84, such that second outlet port 88 is on an opposite side of second valve stem housing 102 from second inlet port 84. This is merely one example configuration, and other configurations are both possible and contemplated.

Manifold housing 80 is typically manufactured from a biocompatible material, e.g., titanium, stainless steel, or biologically inert polymer, and sized, e.g., to accommodate desired flow rates. In some examples, manifold housing 80 is fabricated from a rigid material that is substantially inflexible and unbending, e.g., when fluid is flowing through the manifold housing. In other examples, manifold housing 80 is fabricated from a material that is capable of flexing when fluid is flowing through the manifold. For example, rather than providing a rigid structure to which flexible polymeric tubing can connect, manifold housing 80 may instead be formed from a network of flexible polymeric tubing, e.g., that directly connects to pressurizing units in fluid injection device 16.

Injection manifold 18 can utilize any suitable type of valve for first valve 92 and second valve 94. Example valves that can be used include for example, a ball valve, check valve, gate valve, piston valve, or similar fluid control feature. In one example, each valve can include a pinch valve, which can controllably pinch and release (e.g., compress and depress) a portion of compressible tubing (e.g., a portion of compressible polymeric tubing) to control fluid communication through the tubing. Regardless of the type of valves used within injection manifold 18, each valve can be coupled to a drive mechanism, such as a solenoid or pneumatic actuator within injection device 16, to control opening and closing of the different valves during operation of the injection device. In such examples, one or more processors within fluid injection system 10 may send and receive control signals and/or other information to and from the drive mechanism to control opening and closing of first valve 92 and second valve 94.

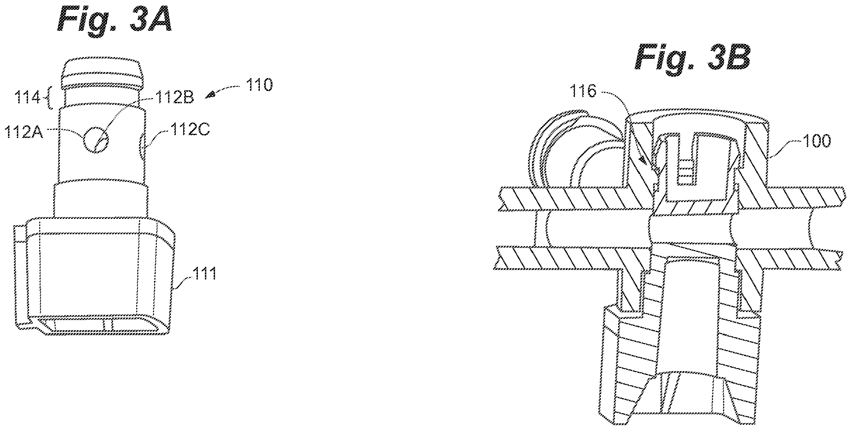

FIGS. 3A and 3B are drawings illustrating one example type of valve that can be used as first valve 92 and/or second valve 94 in injection manifold 18. FIG. 3A illustrates a valve stem 110 that is insertable into a valve stem housing to form a valve unit. FIG. 3B is a cross-sectional illustration of valve stem 110 inserted into a valve stem housing, such as valve stem housing 100 from FIG. 2.

As shown in FIG. 3A, valve stem 110 includes a valve stem handle 111 and a plurality of flow apertures 112A, 112B, 112C extending through the valve stem. Valve stem handle 111 can be grasped to rotate valve stem 110, thereby changing the position of flow apertures 112A, 112B, 112C and, correspondingly, opening and/or closing fluid flow through the different apertures. In some examples, valve stem handle 111 is mated with an electromechanical actuation device associated with fluid injection device 16 to control rotation of the valve stem handle.

Flow apertures 112A, 112B, 112C define openings through which fluid can enter valve stem 110 and pass through the valve stem. Flow aperture 112A is positioned on an opposite side (e.g., 180 degrees) of valve stem 110 from flow aperture 112B, while flow aperture 112C is positioned orthogonally (e.g., 90 degrees) from both flow aperture 112A and 112B. In some examples, an opposite side (e.g., 180 degrees) of valve stem 110 from flow aperture 112C does not have an aperture, providing a sidewall surface of the valve stem that can block fluid flow. Depending on the rotational position of valve stem 110 within valve stem housing 100, fluid may enter the valve stem through one aperture (e.g., flow aperture 112A) and discharge through a different aperture (e.g., flow aperture 112B and/or 112C).

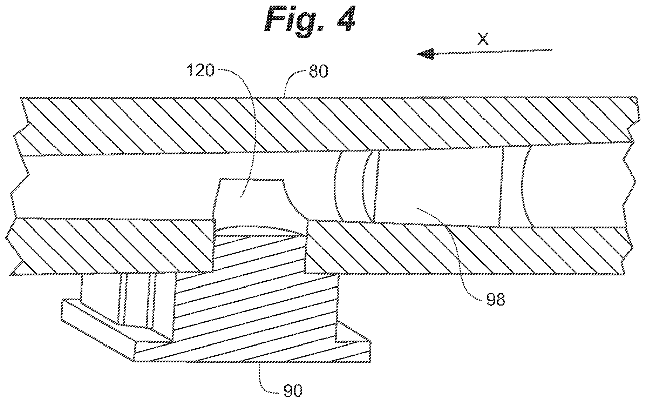

Valve stem housing 100 in FIG. 4B provides a bore that receives valve stem 110. Valve stem 110 can be inserted into valve stem housing 100 by pressing the valve stem axially into the stem housing. In some examples, valve stem 110 is sized larger than a bore of valve stem housing 100 to create an interference (e.g., friction) fit between the valve stem and valve stem housing, which may help prevent fluid leakage during operation of the valve. In addition, mechanical fixation features can secure valve stem 110 in valve stem housing 100, ensuring that the valve stem does not become detached from the valve stem housing during normal operation of the valve. In the example of FIGS. 3A and 3B, valve stem 110 defines a recess 114 that receives a corresponding projection 116 of valve stem housing 100. Upon inserting the valve stem into the valve stem housing, projection 116 can deflect until the projection is coplanar with recess 114, providing a snap latch that frictionally locks the valve stem in the valve stem housing. In other examples, the positions of recess 114 and projection 116 are reversed or a different mechanical fixation is used, and the disclosure is not limited in this respect.

In addition to providing first valve 92 and second valve 94, injection manifold 18 in FIG. 2 also includes pressure sensor 90. Pressure sensor 90 is positioned at least partially, and in some examples fully, within manifold housing 80. This positioning can ensure that the pressure sensor is in physical contact with fluid within manifold housing 80 and, more particularly, bridging portion 96 of the housing, during use of the manifold. FIG. 4 is a cross-sectional illustration of manifold housing 80 illustrating an example configuration by which pressure sensor 90 and dampening device 98 (when used) can be positioned within the housing.

In the example of FIG. 4, manifold housing 80 has a pressure sensor bore 120 extending through an external wall surface of the housing and into an internal flow channel of the housing. Pressure sensor 90 is inserted into pressure sensor bore 120, e.g., such that at least one surface of the pressure sensor is positioned to contact fluid within the internal flow channel of the housing. When so configured, pressure sensor 90 can be secured in pressure sensor bore 120 via friction fit and/or a mechanical fixation element, such as bolts, screws, adhesive, or the like.

In instances in which manifold housing 80 also contains dampening device 98, the dampening device can be secured in the manifold housing using any acceptable techniques. As one example, dampening device 98 can be positioned within an internal flow channel of manifold housing 80 containing pressure sensor 90. For instance, in the example of FIG. 4, dampening device 98 is positioned within a flow channel of manifold housing 80 containing pressure sensor 90, e.g., between first valve 92 and the pressure sensor. To retain dampening device 98 in the flow channel, the flow channel can have a narrowing cross-sectional diameter in the direction of flow toward pressure sensor 90 (e.g., in the X-direction indicated on FIG. 4). When so configured, dampening device 98 can have a reverse taper corresponding to the angle of taper of the flow channel. Upon inserting damping device 98 into the flow channel, the tapered wall surfaces of the flow channel will engage with the reverse taped profile of the dampening device, creating a friction fit that locks the dampening device in the flow channel.

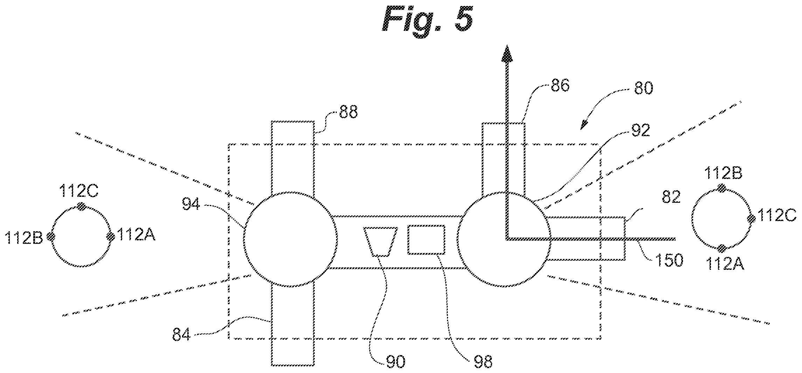

As briefly discussed above, first valve 92 and second valve 94 can assume various operating positions to shield pressure sensor 90 from high pressures generated during contrast injection yet allow that pressure sensor to sense a patient's hemodynamic pressure. FIGS. 5-9 are conceptual illustrations showing different operating positions that can be assumed by first valve 92 and/or second valve 94 to control fluid movement through injection manifold 18. For ease of reference, the operating positions in FIGS. 5-9 are described with respect to valves having three flow apertures 112A, 112B, 112C. It should be appreciated that different valve configurations and operating positions are possible, as discussed herein.

FIG. 5 is a conceptual illustration of first valve 92 and second valve 94 showing an example first operating position in which a first pressurized medical fluid (e.g., diluent) received from first pressurizing unit 20 (FIG. 1) is allowed to flow through manifold housing 80. As shown, a flow of pressurized first medical fluid 150 is received from first pressurizing unit 20 via first inlet port 82. First valve 92 is rotated so the flow of fluid enters the valve stem via flow aperture 112C. The flow of fluid passes through the valve stem via flow aperture 112B and discharges from the manifold housing via first outlet port 86. To block any fluid from second pressurizing unit 24 from flowing through manifold housing 80 in this example, second valve 94 can be rotated so a sidewall of the valve stem that does not have a flow aperture is facing second inlet port 84. Alternatively, if second pressurizing unit 24 is not discharging pressurized fluid, second valve 94 can be rotated to any suitable position while still blocking fluid flow, as no fluid is entering the manifold housing via second inlet port 84 to be blocked.

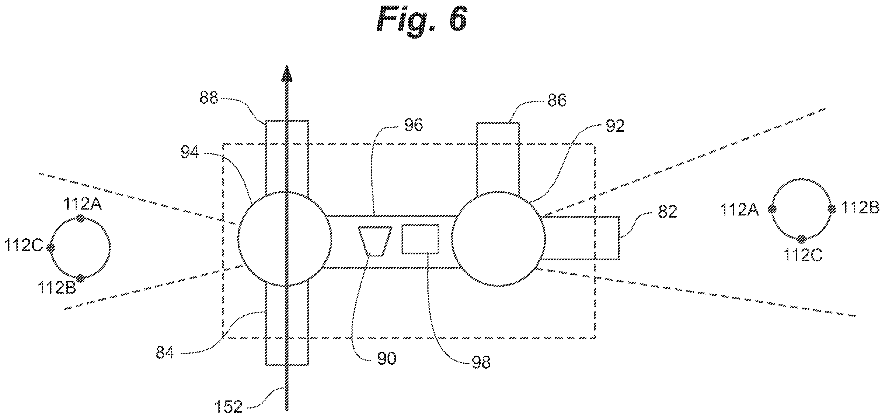

FIG. 6 is a conceptual illustration of first valve 92 and second valve 94 showing an example second operating position in which a second pressurized medical fluid (e.g., contrast media) received from second pressurizing unit 24 (FIG. 2) is allowed to flow through manifold housing 80. As shown, a flow of pressurized second medical fluid 152 is received from second pressurizing unit 24 via second inlet port 84. Second valve 94 is rotated so the flow of fluid enters the valve stem via flow aperture 112B. The flow of fluid passes through the valve stem via flow aperture 112A and discharges from the manifold housing via second outlet port 88. In addition, a sidewall of the valve stem of second valve 94 that does have a flow aperture is positioned facing bridging portion 96. The sidewall of the valve stem can act as a pressure protection barrier to prevent high pressure contrast media from communicating with pressure sensor 90 via bridge portion 96. Further, to block high pressure from the contrast media from entering back into manifold housing 80 via first outlet port 86 and damaging pressure sensor 90, first valve 92 can be rotated so a sidewall of the valve stem that does not have a flow aperture is facing first outlet port 86. This sidewall of the valve can also act as a pressure protection barrier and prevent damage to the pressure sensor.

FIG. 7 is a conceptual illustration of first valve 92 and second valve 94 showing an example third operating position in which neither a first pressurizing medical fluid (e.g., diluent) or a second pressurized medical fluid (e.g., contrast media) are being injected through the manifold housing but in which fluid communication is open between pressure sensor 90 and first outlet port 86. Such an operation position may be useful to determine a patient's hemodynamic pressure. As shown, pressure and/or fluid communicating from a patient via tubing 30 (FIG. 1) is received via first inlet port 82. First valve 92 is rotated so the pressure and/or fluid enters the valve stem via flow aperture 112A. The pressure and/or fluid passes through the valve stem via flow aperture 112C and communicates with pressure sensor 90 within bridging portion 96.

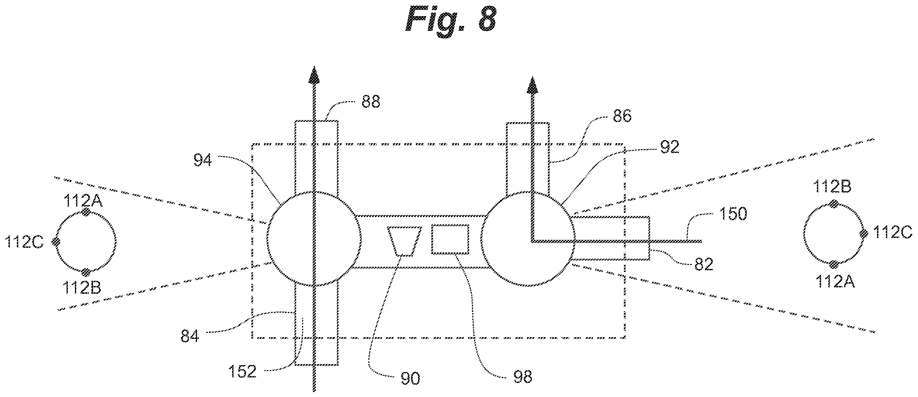

FIG. 8 is a conceptual illustration of first valve 92 and second valve 94 showing an example fourth operating position in which both a first pressurized medical fluid (e.g., diluent) received from first pressurizing unit 20 (FIG. 1) and a second pressurized medical fluid (e.g., contrast media) are allowed to simultaneously flow through manifold housing 80. As shown, a flow of pressurized first medical fluid 150 is received from first pressurizing unit 20 via first inlet port 82. First valve 92 is rotated so the flow of fluid enters the valve stem via flow aperture 112C. The flow of fluid passes through the valve stem via flow aperture 112B and discharges from the manifold housing via first outlet port 86. In addition, a flow of pressurized second medical fluid 152 is received from second pressurizing unit 124 via second inlet port 84. Second valve 94 is rotated so the flow of fluid enters the valve stem via flow aperture 112B. The flow of fluid passes through the valve stem via flow aperture 112A and discharges from the manifold housing via second outlet port 88.

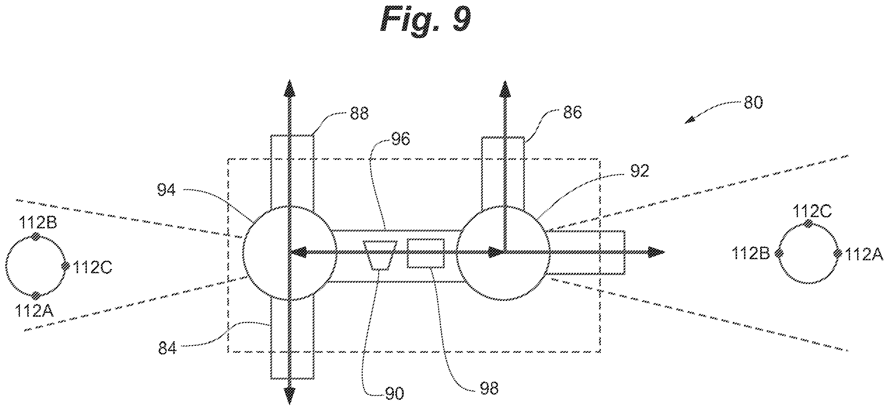

FIG. 9 is a conceptual illustration of first valve 92 and second valve 94 showing an example fifth operating position in which the valves are opened to allow complete fluid communication through manifold housing 80. Such an operating position may be useful, for example, to allow sterilizing gas to pass through the manifold during manufacture, to fill the manifold with fluid and remove air prior to the start of a patient injection procedure, or the like. As shown, first valve 92 is rotated so flow aperture 112A is facing first inlet port 82, flow aperture 112B is facing bridge portion 96, and flow aperture 112C is facing first outlet port 86. In addition, second valve 94 is rotated so flow aperture 112A is facing second inlet port 84, flow aperture 112B is facing second outlet port 88, and flow aperture 112C is facing bridge portion 96.

Various examples have been described. These and other examples are within the scope of the following claims.

## Claims

1. A medical fluid injection manifold (18) comprising:
a manifold housing (80) having a first inlet port (82) configured to receive a first pressurized medical fluid, a second inlet port (84) configured to receive a second pressurized medical fluid, a first outlet port (86) configured to discharge the first pressurized medical fluid, a second outlet port (88) configured to discharge the second pressurized medical fluid and a bridge portion (96);
a pressure sensor (90) configured to measure a hemodynamic pressure of a patient;
**characterized in that** it further comprises: a first valve (92) configured to control fluid flow between the first inlet port (82), the first outlet port (86), and the pressure sensor (90); and
a second valve (94) configured to control fluid flow between the second inlet port (84) and the second outlet port (88),
wherein the bridge portion extends between the first valve (92) and the second valve (94) and the pressure sensor (90) is positioned within the bridge portion (96) of the manifold housing (80; and
wherein the first valve (92) and the second valve (94) have a first operational position in which the first pressurized medical fluid is allowed to flow through the manifold housing (80), a second operational position in which the second pressurized medical fluid is allowed to flow through the manifold housing (80) but the second pressurized medical fluid is blocked from communicating with the pressure sensor (90), and a third operational position in which fluid communication is open between the pressure sensor (90) and the first outlet port (82).

2. The medical fluid injection manifold (18) of claim 1, wherein the pressure sensor (90) comprises a pressure transducer, and further comprising a fluid signal dampening device (98) positioned between the first valve (92) and the pressure sensor (90).

3. The medical fluid injection manifold (18) of claim 2, wherein the fluid signal dampening device (98) comprises a filter.

4. The medical fluid injection manifold (18) of claim 1, further comprising a fluid signal dampening device (98) positioned within the bridge portion (96) of the manifold housing (80) between the first valve (92) and the pressure sensor (90).

5. The medical fluid injection manifold (18) of claim 1, wherein the first outlet port (86) has a smaller diameter than the second outlet port (88).

6. The medical fluid injection manifold (18) of claim 1, wherein the first pressurized medical fluid comprises a diluent and the second pressurized medical fluid comprises a contrast medium.

7. The medical fluid injection manifold (18) of claim 1, wherein the manifold housing (80) is fabricated from a rigid material.

8. The medical fluid injection manifold (18) of claim 1, wherein the manifold housing (80) and pressure sensor (90) positioned therein is removably insertable into a medical fluid injection device (16).

9. A medical fluid injection system (10) comprising:
a medical fluid injection device (16) that includes a first fluid pressurizing unit (20) in fluid communication with a first medical fluid and a second fluid pressurizing unit (24) in fluid communication with a second medical fluid;
a tubing (30) that includes a first fluid lumen (46) configured to convey the first medical fluid from the medical fluid injection device (16) to a patient and a second fluid lumen (48) configured to convey the second medical fluid from the medical fluid injection device (16) to the patient; and
a fluid injection manifold (18) according to any one of claims 1 to 8, wherein the first inlet port (82) of the manifold housing (80) is configured to receive the first pressurized medical fluid from the first fluid pressurizing unit (20) and the second inlet port (84) is configured to receive the second pressurized medical fluid from the second fluid pressurizing unit (24) .

10. The medical fluid injection system (10) of claim 9, wherein the first fluid pressurizing unit (20) comprises a pump, the second fluid pressurizing unit (24) comprises a syringe, and wherein the first medical fluid comprises a diluent and the second medical fluid comprises a contrast medium.

11. The medical fluid injection system (10) of claim 9, wherein the tubing (30) further comprises a junction (50) joining the first fluid lumen (46) and the second fluid lumen (48) into a single lumen configured to be coupled to a patient line, and wherein the junction (50) is configured to be positioned so a distance between the patient and the junction (50) is less than a distance between the junction (50) and the medical fluid injection device (16).

12. A method of measuring the hemodynamic pressure of a patient comprising:
setting a first valve (92) and a second valve (94) of a fluid injection manifold housing (80) of a fluid injection manifold (18) to a first operational position such that a first pressurized medical fluid is injectable through the fluid injection manifold housing (80) while a second pressurized medical fluid is blocked from flowing through the fluid injection manifold housing (80);
setting the first valve (92) and the second valve (94) of the fluid injection manifold housing (80) of the fluid injection manifold (18) to a second operational position such that a second pressurized medical fluid is injectable through the fluid injection manifold housing (80) while the first pressurized medical fluid is blocked from flowing through the fluid injection manifold housing (80);
setting the first valve (92) and the second valve (94) of the fluid injection manifold housing (80) of the fluid injection manifold (18) to a third operational position such that both the first pressurized medical fluid and the second pressurized medical fluid are blocked from flowing through the fluid injection manifold housing (80) but a pressure sensor (90) positioned within the fluid injection manifold (18) between the first valve (92) and the second valve (94) is allowed to communicate with a column of fluid extending from a patient into the fluid injection manifold housing (80).

13. The method of claim 12, wherein the pressure sensor (90) is positioned in a portion of the fluid injection manifold (18) through which fluid can flow and that extends between the first valve (92) and the second valve (94).

14. The method of claim 13, further comprising a fluid signal dampening device (98) positioned in the portion of the fluid injection manifold extending between the first valve (92) and the second valve (94).

15. The method of claim 12, wherein the first pressurized medical fluid comprises a diluent and the second pressurized medical fluid comprises a contrast medium.

## Patentansprüche

1. Einspritz-Verteiler für medizinisches Fluid (18) welcher umfasst:
ein Verteiler-Gehäuse (80) mit einer ersten Einlaßöffnung (82), die ausgestaltet ist, ein erstes, unter Druck stehendes medizinisches Fluid aufzunehmen, einer zweiten Einlaßöffnung (84), die ausgestaltet ist, ein zweites unter Druck stehendes medizinisches Fluid aufzunehmen, einer ersten Auslaßöffnung (86), die ausgestaltet ist, das erste, unter Druck stehende medizinische Fluid auszugeben, einer zweiten Auslaßöffnung (88), die ausgestaltet ist, das zweite, unter Druck stehende medizinische Fluid auszugeben und mit einem Brückenbereich (96);
einen Drucksensor (90), der ausgestaltet ist, einen hämodynamischen Druck eines Patienten zu erfassen;
**dadurch gekennzeichnet, dass** es weiter umfasst:
ein erstes Ventil (92), das ausgestaltet ist, einen Fluidstrom zwischen der ersten Einlaßöffnung (82), der ersten Auslaßöffnung (86), und dem Drucksensor (90) zu steuern; und
ein zweites Ventil (94) das ausgestaltet ist, einen Fluidstrom zwischen der zweiten Einlaßöffnung (84) und der zweiten Auslaßöffnung (88) zu steuern,
worin sich der Brückenbereich zwischen dem ersten Ventil (92) und dem zweiten Ventil (94) erstreckt und der Drucksensor (90) in dem Brückenbereich (96) des Verteiler-Gehäuses (80) angeordnet ist; und
worin das erste Ventil (92) und das zweite Ventil (94) eine erste Betriebs-Position aufweisen, in der das erste unter Druck stehende medizinisches Fluid durch das Verteiler-Gehäuse (80) strömen kann, eine zweite Betriebs-Position, in der das zweite, unter Druck stehende Fluid medizinisches Fluid durch das Verteiler-Gehäuse (80) strömen kann, wobei das zweite unter Druck stehende medizinische Fluid nicht mit dem Drucksensor (90) in Kommunikation steht, und einer dritten Betriebsposition, in der eine Fluid-Verbindung zwischen dem Drucksensor (90) und der ersten Auslaßöffnung (82) offen ist.

2. Einspritz-Verteiler für medizinisches Fluid (18) nach Anspruch 1, worin der Drucksensor (90) einen Druck-Transducer umfasst, und weiter ein Fluid-Signaldämpfungsgerät (98) umfasst, das zwischen dem ersten Ventil (92) und dem Drucksensor (90) angeordnet ist.

3. Einspritz-Verteiler für medizinisches Fluid (18) nach Anspruch 2, worin das Fluid-Signaldämpfungsgerät (98) einen Filter umfasst.

4. Einspritz-Verteiler für medizinisches Fluid (18) nach Anspruch 1, welches weiter ein Fluid-Signaldämpfungsgerät (98) umfasst, das zwischen dem Brückenbereich (96) des Verteiler-Gehäuses (80) zwischen dem ersten Ventil (92) und dem Drucksensor (90) angeordnet ist.

5. Einspritz-Verteiler für medizinisches Fluid (18) nach Anspruch 1, worin die erste Auslaßöffnung (86) einen kleineren Durchmesser aufweist als die zweite Auslaßöffnung (88).

6. Einspritz-Verteiler für medizinisches Fluid (18) nach Anspruch 1, worin das erste unter Druck stehende medizinische Fluid ein Verdünnungsmittel umfasst, und worin das zweite unter Druck stehende medizinische Fluid ein Kontrastmittel umfasst.

7. Einspritz-Verteiler für medizinisches Fluid (18) nach Anspruch 1, worin das Verteiler-Gehäuse (80) aus einem festen Material besteht.

8. Einspritz-Verteiler für medizinisches Fluid (18) nach Anspruch 1, worin das Verteiler-Gehäuse (80) und der darin angeordnete Drucksensor (90) entfernbar in ein Imjektionsgerät für ein medizinisches Fluid (16) einsetzbar ist.

9. Injektionssystem für ein medizinisches Fluid (10) welches umfasst:
ein Injektionsgerät für ein medizinisches Fluid (16), das eine erste Fluid-Druckeinheit (20) umfasst, die in Fluid-Verbindung mit einem ersten medizinischen Fluid steht, und eine zweite Fluid-Druckeinheit (24), die in Fluid-Verbindung mit einem zweiten medizinischen Fluid steht;
ein Rohr (30), das ein erstes Fluid-Lumen (46) umfasst, das ausgestaltet ist, das erste medizinische Fluid von dem Einspritzgerät für medizinisches Fluid (16) zu einem Patienten zu liefern und ein zweites Fluid-Lumen (48), das ausgestaltet ist, das zweite medizinische Fluid von dem Einspritzgerät für medizinisches Fluid (16) zu dem Patienten zu liefern; und
einen Fluideinspritz-Verteiler (18) nach einem der Ansprüche 1 bis 8, worin die erste Einlaßöffnung (82) des Verteiler-Gehäuses (80) ausgestaltet ist, das erste unter Druck stehende medizinische Fluid von der ersten Fluid-Druckeinheit (20) zu erhalten und worin die zweite Einlaßöffnung (84) ausgestaltet ist, das zweite unter Druck stehende medizinische Fluid von der zweiten Fluid-Druckeinheit (24) zu erhalten.

10. Einspritzsystem für medizinisches Fluid (10) nach Anspruch 9, worin die erste Fluid-Druckeinheit (20) eine Pumpe umfasst, worin die zweite Fluid-Druckeinheit (24) eine Spritze umfasst, und worin das erste medizinische Fluid ein Verdünnungsmittel und das zweite medizinische Fluid ein Kontrastmittel umfasst.

11. Einspritzsystem für medizinisches Fluid (10) nach Anspruch 9, worin das Rohr (30) weiter eine Verzweigung (50) umfasst, die das erste Fluid-Lumen (46) und das zweite Fluid-Lumen (48) zu einem einzige Lumen zusammenbringt, das ausgestaltet ist, mit einer Leitung zum Patienten gekoppelt zu werden, und worin die Verbindung (50) ausgestaltet ist, derart angeordnet zu werden, dass eine Entfernung zwischen dem Patienten und der Verbindung (50) weniger als eine Entfernung zwischen der Verbindung (50) und dem Einspritzgerät für medizinisches Fluid (16) ist.

12. Verfahren zur Bestimmung des hämodynamischen Drucks eines Patienten, welches umfasst:
Einstellen eins ersten Ventils (92) und eines zweiten Ventils (94) eines Fluideinspritz-Verteiler-Gehäuses (80) eines Fluideinspritz-Verteilers (18) in eine erste Betriebsposition, so dass ein erstes unter Druck stehendes medizinisches Fluid durch das Fluideinspritz-Verteiler-Gehäuse (80) einspritzbar ist, während ein zweites unter Druck stehendes medizinisches Fluid davon abgehalten wird, durch das Fluideinspritz-Verteiler-Gehäuse (80) zu strömen;
Einstellen des ersten Ventils (92) und des zweiten Ventils (94) des Fluideinspritz-Verteiler-Gehäuses (80) des Fluideinspritz-Verteilers (18) in eine zweite Betriebsposition, so dass ein zweites unter Druck stehendes medizinisches Fluid durch das Fluideinspritz-Verteiler-Gehäuse (80) einspritzbar ist, während das erste unter Druck stehende medizinische Fluid davon abgehalten wird, durch das Fluideinspritz-Verteiler Gehäuse (80) zu strömen;
Einstellen des ersten Ventils (92) und des zweiten Ventils (94) des Fluideinspritz-Verteiler-Gehäuses (80) des Fluideinspritz-Verteilers (18) in eine dritte Betriebsposition, so dass das erste unter Druck stehende medizinische Fluid und das zweite unter Druck stehendes medizinische Fluid davon abgehalten werden, durch das Fluideinspritz-Verteiler-Gehäuse (80) zu strömen, wobei ein Drucksensor (90), der in dem Fluideinspritz-Verteiler (18) zwischen dem ersten Ventil (92) und dem zweiten Ventil (94) angeordnet ist, mit einer Fluid-Säule in Verbindung steht, die sich von einem Patienten in das Fluideinspritz-Verteiler-Gehäuse (80) erstreckt.

13. Verfahren nach Anspruch 12, worin der Drucksensor (90) in einem Bereich des Fluideinspritz-Verteilers (18) angeordnet ist, durch den Fluid strömen kann und der sich zwischen dem ersten Ventil (92) und dem zweiten Ventil (94) erstreckt.

14. Verfahren nach Anspruch 13, welches weiter ein Fluid-Signaldämpfungsgerät (98) umfasst, das in dem Bereich des Fluideinspritz-Verteilers angeordnet ist, der sich zwischen dem ersten Ventil (92) und dem zweiten Ventil (94) erstreckt.

15. Verfahren nach Anspruch 12, worin das erste unter Druck stehende medizinische Fluid ein Verdünnungsmittel umfasst und das zweite unter Druck stehende medizinische Fluid ein Kontrastmittel umfasst.

## Revendications

1. Collecteur d'injection de fluide médical (18) comprenant :
une enceinte de collecteur (80) qui possède un premier orifice d'admission (82) configuré pour recevoir un premier fluide médical pressurisé, un second orifice d'admission (84) configuré pour recevoir un second fluide mécanique pressurisé, un premier orifice d'évacuation (86) configuré pour évacuer le premier fluide médical pressurisé, un second orifice d'évacuation (88) configuré pour évacuer le second fluide médical pressurisé et une partie de jonction (96) ;
un capteur de pression (90) configuré pour mesurer une pression hémodynamique d'un patient ;
**caractérisé en ce qu'**il comprend en outre : une première soupape (92) configurée pour réguler l'écoulement de fluide entre le premier orifice d'admission (82), le premier orifice d'évacuation (86), et le capteur de pression (90) ; et
une seconde soupape (94) configurée pour réguler l'écoulement de fluide entre le second orifice d'admission (84) et le second orifice d'évacuation (88),
dans lequel la partie de jonction s'étend entre la première soupape (92) et la seconde soupape (94) et le capteur de pression (90) est positionné dans la partie de jonction (96) de l'enceinte de collecteur (80) ; et
dans lequel la première soupape (92) et la seconde soupape (94) possèdent une première position de fonctionnement dans laquelle le premier fluide médical pressurisé peut circuler dans l'enceinte de collecteur (80), une seconde position de fonctionnement dans laquelle le second fluide médical pressurisé peut circuler dans l'enceinte de collecteur (80), mais le second fluide médical pressurisé ne peut pas communiquer avec le capteur de pression (90), et une troisième position de fonctionnement dans laquelle la communication de fluide est ouverte entre le capteur de pression (90) et le premier orifice d'évacuation (82).

2. Collecteur d'injection de fluide médical (18) selon la revendication 1, dans lequel le capteur de pression (90) comprend un transducteur de pression, et comprenant en outre un dispositif d'amortissement de signal de fluide (98) positionné entre la première soupape (92) et le capteur de pression (90).

3. Collecteur d'injection de fluide médical (18) selon la revendication 2, dans lequel le dispositif d'amortissement de signal de fluide (98) comprend un filtre.

4. Collecteur d'injection de fluide médical (18) selon la revendication 1, comprenant en outre un dispositif d'amortissement de signal de fluide (98) positionné dans la partie de jonction (96) de l'enceinte de collecteur (80) entre la première soupape (92) et le capteur de pression (90).

5. Collecteur d'injection de fluide médical (18) selon la revendication 1, dans lequel le premier orifice d'évacuation (86) possède un diamètre inférieur au second orifice d'évacuation (88).

6. Collecteur d'injection de fluide médical (18) selon la revendication 1, dans lequel le premier fluide médical pressurisé comprend un diluant le second fluide médical pressurisé comprend un milieu de contraste.

7. Collecteur d'injection de fluide médical (18) selon la revendication 1, dans lequel l'enceinte de collecteur (80) est fabriquée avec un matériau rigide.

8. Collecteur d'injection de fluide médical (18) selon la revendication 1, dans lequel l'enceinte de collecteur (80) et le capteur de pression (90) positionné à l'intérieur peuvent être insérés de manière amovible dans un dispositif d'injection de fluide médical (16).

9. Système d'injection de fluide médical (10) comprenant :
un dispositif d'injection de fluide médical (16) qui comprend une première unité de pressurisation de fluide (20) en communication de fluide avec un premier fluide médical, et une seconde unité de pressurisation de fluide (24) en communication de fluide avec un second fluide médical ;
un tube (30) qui comprend une première lumière de fluide (46) configurée pour acheminer le premier fluide médical du dispositif d'injection de fluide médical (16) vers un patient, et une seconde lumière de fluide (48) configurée pour acheminer le second fluide médical du dispositif d'injection de fluide médical (16) vers le patient ; et
un collecteur d'injection de fluide (18) selon l'une quelconque des revendications 1 à 8, dans lequel le premier orifice d'admission (82) de l'enceinte de collecteur (80) est configuré pour recevoir le premier fluide médical pressurisé de la part de la première unité de pressurisation de fluide (20), et le second orifice d'admission (84) est configuré pour recevoir le second fluide médical pressurisé de la part de la seconde unité de pressurisation de fluide (24).

10. Système d'injection de fluide médical (10) selon la revendication 9, dans lequel la première unité de pressurisation de fluide (20) comprend une pompe, la seconde unité de pressurisation de fluide (24) comprend une seringue, et dans lequel le premier fluide médical comprend un diluant et le second fluide médical comprend un milieu de contraste.

11. Système d'injection de fluide médical (10) selon la revendication 9, dans lequel le tube (30) comprend en outre une jonction (50) qui joint la première lumière de fluide (46) et la seconde lumière de fluide (48) dans une seule lumière configurée pour être reliée à une ligne de patient, et dans lequel la jonction (50) est configurée pour être positionnée de sorte qu'une distance entre le patient et la jonction (50) soit inférieure à une distance entre la jonction (50) et le dispositif d'injection de fluide médical (16).

12. Procédé de mesure de la pression hémodynamique d'un patient, comprenant :
le réglage d'une première soupape (92) et d'une seconde soupape (94) d'une enceinte de collecteur d'injection de fluide (80) d'un collecteur d'injection de fluide (18) dans une première position de fonctionnement de sorte qu'un premier fluide médical pressurisé soit injectable dans l'enceinte de collecteur d'injection de fluide (80) pendant qu'un second fluide médical pressurisé ne peut circuler dans l'enceinte de collecteur d'injection de fluide (80) ;
le réglage de la première soupape (92) et de la seconde soupape (94) de l'enceinte de collecteur d'injection de fluide (80) du collecteur d'injection de fluide (18) dans une seconde position de fonctionnement de sorte qu'un second fluide médical pressurisé soit injectable dans l'enceinte de collecteur d'injection de fluide (80) pendant que le premier fluide médical pressurisé ne peut circuler dans l'enceinte de collecteur d'injection de fluide (80) ;
le réglage de la première soupape (92) et de la seconde soupape (94) de l'enceinte de collecteur d'injection de fluide (80) du collecteur d'injection de fluide (18) dans une troisième position de fonctionnement de sorte que le premier fluide médical pressurisé et le second fluide médical pressurisé ne puissent pas circuler dans l'enceinte de collecteur d'injection de fluide (80), mais qu'un capteur de pression (90) positionné dans le collecteur d'injection de fluide (18) entre la première soupape (92) et la seconde soupape (94) puisse communiquer avec une colonne de fluide qui s'étend entre un patient et l'enceinte de collecteur d'injection de fluide (80).

13. Procédé selon la revendication 12, dans lequel le capteur de pression (90) est positionné dans une partie du collecteur d'injection de fluide (18) dans laquelle le fluide peur circuler, et qui s'étend entre la première soupape (92) et la seconde soupape (94).

14. Procédé selon la revendication 13, comprenant en outre un dispositif d'amortissement de signal de fluide (98) positionné dans la partie du collecteur d'injection de fluide qui s'étend entre la première soupape (92) et la seconde soupape (94).

15. Procédé selon la revendication 12, dans lequel le premier fluide médical pressurisé comprend un diluant et le second fluide médical pressurisé comprend un milieu de contraste.
